# EUROPEAN PATENT SPECIFICATION

(11) **EP 2 140 256 B1**
(45) Date of publication and mention of the grant of the patent: **20.06.2012**
(21) Application number: 08737943.4
(22) Date of filing: 22.04.2008
(51) Int. Cl.: G01N 27/327

(54) **BIOSENSOR CHIP**
BIOSENSORCHIP
PUCE DE BIOCAPTEUR

(30) Priority: 27.04.2007 EP 07107118
(43) Date of publication of application: 06.01.2010
(73) Proprietor: NXP B.V., 5656 AG Eindhoven (NL)
(72) Inventor: GARCIA TELLO, Pablo, A-1102 Vienna (AT); GRIDELET, Evelyne, A-1102 Vienna (AT); WIDDERSHOVEN, Franciscus, A-1102 Vienna (AT)
(74) Representative: Williamson, Paul Lewis
(86) International application number: PCT/IB2008/051538
(87) International publication number: WO 2008/132656

(56) References cited:
- DE-A1-102004 058 064
- WITVROUW A ET AL: "WHY CMOS-INTEGRATED TRANSDUCERS? A REVIEW" MICROSYSTEM TECHNOLOGIES, BERLIN, DE, vol. 6, no. 5, 1 August 2000 (2000-08-01), pages 192-199, XP001050374 ISSN: 0946-7076
- HOFMANN F ET AL: "PASSIVE DNA SENSOR WITH GOLD ELECTRODES FABRICATED IN A CMOS BACKEND PROCESS" PROCEEDINGS OF THE EUROPEAN SOLID STATE DEVICE RESEARCHCONFERENCE (ESSDERC), EDITION FRONTIERES, GIL-SUR-YVETTE, FR, 24 September 2002 (2002-09-24), pages 487-490, XP008033777
- REIMER K ET AL: "FABRICATION OF ELECTRODE ARRAYS IN THE QUARTER MICRON REGIME FOR BIOTECHNOLOGICAL APPLICATIONS" SENSORS AND ACTUATORS A, ELSEVIER SEQUOIA S.A., LAUSANNE, CH, vol. A46, no. 1/03, 1 January 1995 (1995-01-01), pages 66-70, XP000514180 ISSN: 0924-4247
- THEWES R ET AL: "A CMOS medium density DNA microarray with electronic readout" MATERIALS RESEARCH SOCIETY SYMPOSIUM PROCEEDINGS - MATERIALS, INTEGRATION AND TECHNOLOGY FOR MONOLITHIC INSTRUMENTS 2005 MATERIALS RESEARCH SOCIETY US, vol. 869, 2005, pages 91-101, XP008097798
- HOFMANN F ET AL: "Technology aspects of a CMOS Neuro-Sensor: Back End Process and Packaging" EUROPEAN SOLID-STATE DEVICE RESEARCH, 2003 33RD CONFERENCE ON. ESSDERC '03 SEPT. 16-18, 2003, PISCATAWAY, NJ, USA,IEEE, 16 September 2003 (2003-09-16), pages 167-170, XP010676620 ISBN: 978-0-7803-7999-2

## Description

### FIELD OF THE INVENTION

The invention relates to a biosensor chip.

### BACKGROUND OF THE INVENTION

A biosensor may be denoted as a device that may be used for the detection of an analyte that combines a biological component with a physicochemical or physical detector component.

For instance, a biosensor may be based on the phenomenon that capture molecules immobilized on a surface of a biosensor may selectively hybridize with target molecules in a fluidic sample, for instance when an antibody-binding fragment of an antibody or the sequence of a DNA single strand as a capture molecule fits to a corresponding sequence or structure of a target molecule. When such hybridization or sensor events occur at the sensor surface, this may change the electrical properties of the surface, which electrical properties may be detected as the sensor event.

WO 2005/106478 discloses a method for functionalizing biosensors, particularly those based on semiconductor chips mounted on a finished processed wafer, provided with sensor fields placed thereupon, which may be arranged in an array, and for carrying out a functionalization, for example, with organic molecules such as nucleic acids like DNA, RNA and PNA or with their derivatives, proteins, sugar molecules or antibodies.

Conventional biosensor chips usually have a sensing surface being arranged in the Front End of the Line portion of a semiconductor structure, that is to say are located spatially close to integrated semiconductor members such as detection transistors. In other words, in such conventional approaches, a sensor active surface is arranged directly on top of a processed semiconductor structure.

Both Witvrouw et Al: "Why CMOS-Integrated Transducer? A Review", Microsystem Technologies, vol. 6, no. 5, 1 August 2000 (2000-08-01) pages 192-199, ISSN: 0946-7076 and and Hofmann et Al.: "Passive DNA sensor with Gold Electrodes Fabricated in a CMOS Backend Process", Proceedings of ESSDERC 2002, pages 487-490, disclose biosensor chips with a sensor active region in the Back End of the Line Portion.

### OBJECT AND SUMMARY OF THE INVENTION

It is an object of the invention to provide a robust biosensor.

According to the present invention, a biosensor chip for detecting biological particles according to claim 1 is provided.

The term "Back End of the Line" (BEOL) or "Back End of the Line portion" denotes a portion of an integrated circuit fabrication where active components (transistors, resistors, etc.) are interconnected with wiring on the wafer. BEOL generally begins when a first layer of metal is deposited on the processed wafer. It includes contacts, insulator, metal levels, and bonding sites for chip-to-package connections. Thus, each structural component of an integrated circuit which is out of direct contact with the processed semiconductor substrate is considered to belong to the BEOL.

In contrast to this, the term "Front End of the Line" (FEOL) or "Front End of the Line portion" denotes a first portion of an integrated circuit fabrication where the individual devices (transistors, resistors, etc.) are patterned in the semiconductor. FEOL generally covers everything up to (but not including) the deposition of metal layers. Thus, each structural component of an integrated circuit which is part of the processed semiconductor substrate is considered to belong to the FEOL.

In other words, the Back End of the Line portion is located directly on top of the Front End of the Line portion (in a spatial direction which corresponds to the manufacturing procedure).

The term "biosensor" denotes any device that may be used for the detection of an analyte comprising biological molecules such as DNA, RNA, proteins, enzymes, cells, bacteria, virus, etc. A biosensor combines a biological component (for instance capture molecules at a sensor active surface capable of detecting molecules) with a physicochemical or physical detector component (for instance a capacitor having a capacitance which is modifiable by a sensor event, or a layer having a redox potential which is modifiable by a sensor event, or a field effect transistor having a threshold voltage or a channel conductivity which is modifiable by a sensor event).

The term "biosensor chip" denotes that a biosensor is formed as an integrated circuit, that is to say as an electronic chip, particularly in semiconductor technology, more particularly in silicon semiconductor technology, still more particularly in CMOS technology. A monolithically integrated biosensor chip has the property of very small dimensions thanks to the use of micro-processing technology, and may therefore have a large spatial resolution and a high signal-to-noise ratio particularly when the dimensions of the biosensor chip or more precisely of components thereof approach or reach the order of magnitude of the dimensions ofbiomolecules.

The term "biological particles" denotes any particles which play a significant role in biology or in biological or biochemical procedures, such as genes, DNA, RNA, proteins, enzymes, cells, bacteria, virus, etc.

The term "sensor active region" denotes an exposed region of a sensor that may be brought in interaction with a fluidic sample so that a detection event may occur in the sensor active region. In other words, the sensor active region is the actual sensitive area of a sensor device, in which area such processes take place, which form the basis of the sensing.

The term "substrate" denotes any suitable material, such as a semiconductor, glass, plastic, etc. The term "substrate" is used to define generally the elements for layers that underlie and/or overlie a layer or portions of interest, such as a base on which a layer is formed, for example a semiconductor wafer such as a silicon wafer or silicon chip.

The term "fluidic sample" denotes any subset of the phases of matter. Such fluids may include liquids or gases. Examples for fluidic samples are DNA containing fluids, blood, interstitial fluid in subcutaneous tissue, muscle or brain tissue, urine or other body fluids. For instance, the fluidic sample may be a biological substance. Such a substance may comprise proteins, polypeptides, nucleic acids, DNA strands, etc.

According to the present invention, a monolithically integrated biosensor is provided in an electronic chip architecture comprising a (semiconductor) substrate in which first electronic components of the biosensor chip are formed in the Front End of the Line portion. Above the Front end of the Line portion, a second stack of further layers and structures is provided as the Back end of the Line portion. According to the invention, the sensor active region is provided in the Back End of the Line portion. BEOL processing of a particle-sensitive biosensor probe is advantageous due to a spatial separation between actual detection event (performed in the BEOL on the particle-sensitive biosensor probe) and detection signal evaluation (performed in integrated electronic components formed in the FEOL, such as a field effect transistor). Such architecture is particularly advantageous when the nanoelectrodes serving as the sensor active region can be manufactured sufficiently small. For example, such nanoelectrodes can be arranged in the FEOL with dimensions of 250 nm, 130 nm or less, and may for instance be realized as sensing pockets having dimensions close to dimensions of biological molecules to be detected. This may allow obtaining a significant improvement of the signal-to-noise ratio.

A specific advantage of using a BEOL portion for processing a sensor active region is that liquid components (such as an aqueous solution) of a fluidic sample may be brought in interaction with the BEOL layer and are properly separated by the BEOL stack from the below arranged FEOL stack so that there is no danger that FEOL components such as a gate region of a field effect transistor are contaminated or harmed by a fluidic liquid sample. Therefore, by performing the sensor event in the BEOL, it is possible to reliably decouple/isolate the liquid components from the microelectronic detection members provided below the BEOL layer in the FEOL layer. Copper material which is used in standard BEOL procedures has advantageous properties to serve as BEOL electrodes which may be connected to a buried FEOL transistor.

Therefore, according to the present invention, a biosensor is provided having a sensor active surface in the BEOL layer, not in the FEOL layer. In contrast to conventional biosensors having a sensor active surface in the FEOL region and consequently suffering from an undesired interaction between a fluidic sample to be analyzed and semiconductor structures, biosensors of the invention are more robust and allow to be employed under humid and harsh conditions due to the spatial decoupling between sensor active surface (in the BEOL) and a control or sensing electronics (in the FEOL).

For instance, on a copper nanoelectrode, a self-assembled monolayer (SAM) may be provided which may be specifically designed to attach capture molecules such as antibodies. The copper electrode may then serve, in combination with a second electrode which can be another metallization layer of the semiconductor layer sequence or which can be a counter electrode that may be provided apart from the semiconductor layer sequence, as a capacitor. Sensor events (such as hybridization events between capture molecules immobilized on the SAM layer and target molecules in the sample) then modify the value of the capacitance of the capacitor. However, alternatives to a capacitance-based sensing technology are possible, for instance when a reaction at the sensor active surface modifies the electrochemical properties such as the redox potential at a surface of the sensor. This may be sensed by, for instance, a field effect transistor as a characteristic change of the threshold voltage or the source/drain current.

Therefore, with advanced CMOS technologies approaching the size of interesting biomolecules that are involved in processes related to disease diagnostics and monitoring, embodiments of the invention enable to use such advanced CMOS technology as very accurate detectors, particularly for single molecule detection. The nanoelectrodes allow the detection of molecules related to certain diseases (for instance cancer) at the single molecular level. The single molecular range of detection is achieved due to the small dimensions of the electrodes that are manufactured in the BEOL with advanced CMOS technology.

The processing of such nanoelectrodes may involve etch processes and/or chemical mechanical polishing (CMP) techniques, thereby allowing to manufacture a cheap and reliable biosensor which may be appropriate for volume production of single molecular biosensors. Thus, back end processing for a biosensing device is made possible, producing nanoelectrodes for use with the CMOS-based sensor technology, and in particular with procedures involving polishing and/or etching.

In contrast to conventional approaches performing pure front end processing to fabricate CMOS-based biosensors, thereby attaching biomolecules directly to a polysilicon or other material with which the gate of a transistor is fabricated (see traditional concepts as ISFET), the present invention provides the sensor active region in the BEOL, thereby significantly increasing accuracy of the device.

In the context of back end processing for a biosensing device, CMOS technology is used as a strong driver to get generic and highly sensitive biosensing platforms that can be aimed to the point of care disease diagnostic and monitoring. An exemplary application of such a device may be the capturing of an antibody inside of a sensing pocket, to be able to detect its presence with the help of a transistor associated to such a sense pocket. The detection may be done noticing changes in the capacitance created by the presence of the antibody and associated to changes in the drain current of the transistor.

Changes in capacitance may be produced in a sense pocket depending on the presence/absence of a biomolecule (for instance in the context of an antibody-antigen reaction), and this being performed selectively in the back end. The implementation of a biosensor chip based on a simple change in capacitance in the presence of a sensor event in the sensing pocket (for instance on a surface of (nano-)electrodes), allows to provide biosamples which are label-free. Attaching (fluorescence) labels to DNA or other components of a biosensor involves additional effort which is made dispensible by the invention. However, other embodiments of the invention may also involve label-based detection techniques, for instance when reading out sensor events optically (for example fluorescence detection).

By manufacturing a biosensor array having a plurality of biosensor chips integrated in a single common substrate, the accuracy or sensitivity is further improved by such a massive parallel approach. For instance, a large number of nanoelectrodes each connected to an assigned access transistor or readout transistor may be provided, wherein single molecule detection events may occur at each of the nanoelectrodes. Alternatively, the performance of a plurality of sensor events at each nanoelectrode is possible. However, with such a multiple nanoelectrode approach, it is possible to get individual sensor information from each of the nanoelectrodes, thereby significantly improving accuracy.

The electronic device may also be adapted as a biosensor array, that is a configuration of a (large) plurality of biosensor devices of the aforementioned type. In such a biosensor array, the biosensor cells may be arranged in a matrix-like manner and may be controlled via bit lines and word lines with transistors serving as switches to get or prevent access to desired individual biosensor cells. The multiple biosensor cells may be monolithically integrated in a common (for instance silicon) substrate.

According to the invention, an exposed surface of the sensor active region has a dimension of at most 1.6 times, particularly of at most 1.1 times, more particularly of at most 0.7 times, of a minimum lithographic feature size of a CMOS process applied for manufacturing the biosensor chip. Particularly, a biosensor is provided that has a bio-sensitive part made at the surface of a Back End of the Line portion of an advanced CMOS process with copper interconnect, where the diameter of the exposed copper surface is equal to or smaller than 1.6 times the minimum lithographic feature size of the smallest copper via holes of the corresponding CMOS process. A value slightly less than 1 (for instance down to about 0.7) may correspond to sub-feature size holes made by adding minor additional processing steps, or by applying a first-metal feature size. This would require some additional processing steps or more stringent control over more demanding standard CMOS steps (for instance in case of applying first-metal feature size). Even smaller values can be made in principle, but would require extensive additional processing effort. Furthermore, they would lead to a significantly reduced fraction of sensitive area of a biosensor cell. Also, the sensitivity of the sensor would not improve significantly by decreasing the radius even more because the total capacitance of the nanoelectrode sensor node would be limited by parasitic capacitances anyway. To be able to really benefit from smaller nano-electrode radii it would be necessary to decrease the dimensions of the transistors and interconnect layers as well, that is to say stepping to the next CMOS node.

The sensor active region is arranged at an upper surface of a Back End of the Line of the biosensor chip. The term "upper" refers to the order according to which the biosensor device is manufactured (so that the FEOL forms a lower portion and the BEOL forms an upper portion of the biosensor device). Therefore, the sensor active region is exposed to a surface of the biosensor chip that may be brought in an interaction with a fluidic sample to be analyzed. Particularly, a stack of a plurality of layers, involving a plurality of electrically conductive connection elements (such as vias and/or metallization structures) is provided to separate the sensor active region from a readout member (such as a field effect transistor provided in the substrate) spatially, but to couple the sensor active region to the readout member electronically. This allows to properly space the Front End of the Line components which may be degraded by a fluidic or liquid sample from the sensor active surface in the BEOL which may be less prone to degradation by liquids.

The biosensor chip comprises at least one intermediate metallization structure (usually a plurality of intermediate metallization structures) in the Back End of the Line, wherein the sensor active region is electrically coupled to a Front End of the Line of the biosensor chip via the at least one intermediate metallization structure. Particularly, it is possible to provide a plurality of such intermediate metallization structures in multiple layers to provide a reliable spatial separation between FEOL and BEOL. However, such metallization structures which are made of electrically properly conducting copper material may allow for a low ohmic electric coupling between sensor active surface and the lower laying evaluation circuit, such as a transistor.

The biosensor chip may comprise a capacitor structure at least partially formed in the Back End of the Line and arranged such that a capacity value of the capacitor is influencable by a detection event in the sensor active region. Since biomolecules may have dielectric properties, the value of the permittivity of the capacitor may be selectively changed in the presence of such molecules and therefore selectively in the presence of sensor events. One electrode or capacitor plate of such a capacitor may be formed by the metallization electrode forming (part of) the sensor active region itself, and the other capacitor plate may be another electrically conductive structure of the integrated biosensor chip, or may alternatively be a counter electrode provided apart from the layer sequence forming the biosensor chip. For instance, such a counter electrode may be immersed in a solution/fluidic sample to be analyzed. However, it is possible to implement alternative detection schemes which do not include a capacitor, for instance a direct impact of a sensor event on the voltage acting on a gate region of a transistor, or the like.

The biosensor may comprise a switch transistor structure formed in the Front End of the Line and electrically coupled to the sensor active region. Such a switch transistor may be a field effect transistor realized as an n-MOSFET or a p-MOSFET. The sensor active surface may be electrically coupled to one of the source/drain regions of such a switch transistor structure, so that a readout voltage applied to the gate of the transistor may result in a source/drain current which depends on the presence or absence (and also on the amount) of the particles of the fluidic sample, since this may have an impact on the voltage of the capacitor which may be transferred to one of the source/drain regions. Alternatively, such a voltage may directly act on the gate region of a MOSFET, thereby changing the threshold voltage or changing the value of a current flowing between source and drain when a voltage is applied in between.

The biosensor may comprise one capture molecule (or a plurality of capture molecules) arranged (for instance immobilized) at a surface of the sensor active region and adapted for interacting with the biological particles (for instance adapted for hybridizing). More particularly, it may be sufficient to have a single capture molecule arranged at each of the nanoelectrodes, since the implementation of modem CMOS procedures in the Front End of the Line may allow to manufacture the nanoelectrodes with dimensions which are in the same order of magnitude as, for instance, antibodies. However, it is also possible to provide a plurality of capture molecules at each nanoelectrode.

The sensor active region comprises a nanoelectrode, the dimensions of the electrode being in the order of magnitude of nanometers, for instance may be less than 300 nm, for instance may be less or equal than 250 nm, or may be less or equal than 130 nm. The smaller the nanoelectrodes, the more sensitive the resulting sensor pocket.

The nanoelectrode comprises copper material, and may in particular comprise copper material being covered by a self-assembled monolayer (SAM). These materials may serve as oxidation protection layers or as barrier layers or for enabling bonding of capture molecules, thereby allowing to implement the relative sensitive material copper which is highly appropriate due to its high electrical conductivity and compliance with procedural requirements. Copper material has chemically similar properties to gold which is conventionally used in biosensing, but which has significant disadvantages because it diffused rapidly into many materials used in silicon process technology, thereby deteriorating the IC's performance, it is difficult to etch, and gold residues are hard to remove in cleaning steps. The biosensor may comprise an electrically insulating layer forming part of a surface of the biosensor chip and having a recess, wherein an exposed surface of the sensor active region is provided as a sensing pocket in the recess. By providing sensing pockets, shielded and defined regions may be formed in which a sensor event may take place. In the bottom of the recess, the nanoelectrode may be provided with small dimensions, so that a single or a few capture molecules may be arranged in a mechanically protected manner in such sensing pockets. Therefore, the biosensor chip may be used even under harsh conditions.

The biosensor chip is manufactured in CMOS technology. CMOS technology, particularly the latest generations thereof, allow to manufacture structures with very small dimensions so that (spatial) accuracy of the device will be improved by implementing CMOS technology particularly in the Front End of the Line. A CMOS process may be a preferred choice. A BiCMOS process in fact is a CMOS process with some additional processing steps to add bipolar transistors. The same holds for CMOS processes with other embedded options like embedded flask, embedded DRAM, etc. In particular this may be relevant because the presence of an option often provides opportunities to use additional materials that come with the options "at zero cost". For instance, an appropriate high-k material (an insulating material with a high dielectric constant, for example aluminium-oxide) that comes with an embedded DRAM process can be used "at zero cost" to cover the copper surface of the nanoelectrodes with a protective dielectric layer on which, subsequently, a SAM can be deposited (the function of the SAM would be to "functionalize" that sensor surface, for instance to be able to attach capture probe molecules).

The biosensor device is monolithically integrated in a semiconductor substrate, particularly comprising one of the group consisting of a group IV semiconductor (such as silicon or germanium), and a group III-group V semiconductor (such as gallium arsenide).

At the end of a method of manufacturing a biosensor chip, it is possible to form an electrically insulating layer forming part of a surface of the biosensor chip and having a recess, wherein an exposed surface of the sensor active region is provided as a sensing pocket in the recess. In such an embodiment, it may be advantageous to manufacture such a device using an etching procedure for exposing the sensor active surface. However, it may be even more advantageous to manufacture such a layer sequence having an exposed sensor active surface using chemical mechanical polishing (CMP), since the result may allow to have a miniature nanoelectrode with an essentially planar surface.

The biosensor chip or microfluidic device may be or may be part of a sensor device, a sensor readout device, a lab-on-chip, an electrophoresis device, a sample transport device, a sample mix device, a sample washing device, a sample purification device, a sample amplification device, a sample extraction device or a hybridization analysis device. Particularly, the biosensor or microfluidic device may be implemented in any kind of life science apparatus.

For any method step, any conventional procedure as known from semiconductor technology may be implemented. Forming layers or components may include deposition techniques like CVD (chemical vapour deposition), PECVD (plasma enhanced chemical vapour deposition), ALD (atomic layer deposition), or sputtering. Removing layers or components may include etching techniques like wet etching, plasma etching, etc., as well as patterning techniques like optical lithography, UV lithography, electron beam lithography, etc.

The biosensor may be formed on a purely crystalline silicon wafer or on an SOI wafer (Silicon On Insulator).

The aspects defined above and further aspects of the invention are apparent from the examples of embodiment to be described hereinafter and are explained with reference to these examples of embodiment.

### BRIEF DESCRIPTION OF THE DRAWINGS

The invention will be described in more detail hereinafter with reference to examples of embodiment but to which the invention is not limited.
Fig. 1, Fig. 2, Fig. 14 and Fig. 15 show biosensor chips according to exemplary embodiments of the invention.
Fig. 3 to Fig. 11 show layer sequences obtained during the manufacture of biosensor chips according to exemplary embodiments of the invention.
Fig. 12 and Fig. 13 show diagrams illustrating why CMOS technology is specifically appropriate for manufacturing biosensor chips according to exemplary embodiments of the invention.

### DESCRIPTION OF EMBODIMENTS

The illustration in the drawing is schematical. In different drawings, similar or identical elements are provided with the same reference signs.

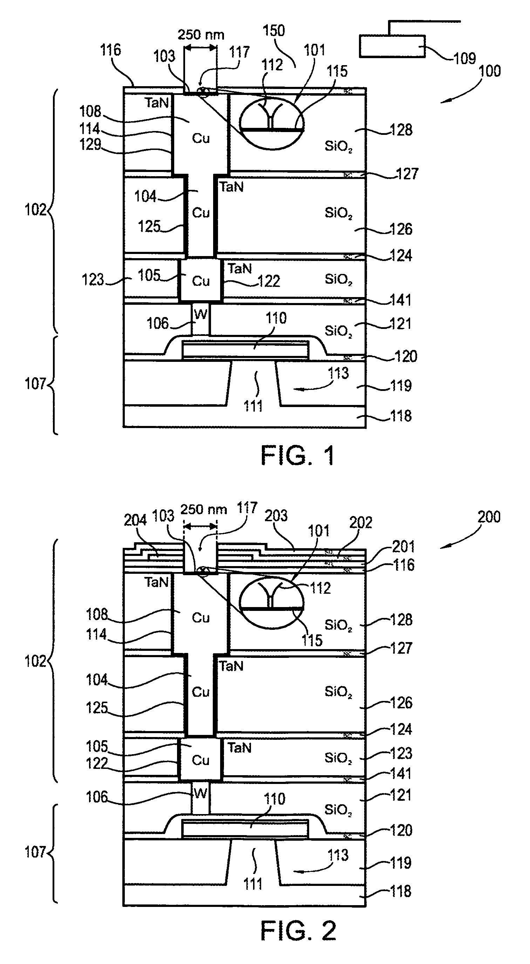

In the following, referring to Fig. 1, a biosensor chip 100 according to an embodiment of the invention will be explained.

The biosensor chip 100 is adapted for detecting biological particles (such as antigens, not shown in the figure) and comprises a sensor active region 101 being sensitive for the biological particles and being arranged on top of a Back End of the Line portion 102 of the biosensor chip 100. More particularly, the sensor active region 101 is arranged at an upper surface 103 of the BEOL region 102 of the biosensor chip 100.

A plurality of intermediate metallization structures 104 to 106 in the BEOL portion 102 are provided so that the sensor active region 101 is electrically coupled to a Front End of the Line portion 107 of the biosensor chip 100 via the plurality of intermediate metallization structures 104 to 106.

More particularly, a nanoelectrode 108 forming part of the sensor active region 101 is electrically coupled via the plurality of intermediate metallization structures 104 to 106 to a field effect transistor 113 integrated in the FEOL region 107.

A capacitor structure is partially formed in the Back End of the Line portion 102 and is arranged such that a capacitance value of the capacitor is influencable by a detection event at the sensor active region 101 (that is by a binding of antigens (not shown) to an antibody 112 immobilized on the surface 103 of the sensor active region 101), since such a detection event may have an impact on the value of the permittivity in a sensor pocket 117. More particularly, a first electrode of such a capacitor is formed by the copper layer 108, and a second electrode of this capacitor is formed by an electrolyte 150, connected by a counter electrode 109 which is, in the present embodiment, provided apart from the monolithically integrated layer sequence 100. Alternatively, it is possible to integrate an electrically conductive structure forming the second electrode of the capacitor in the layer stack. Such an embodiment is shown in Fig. 2 and will be described below in more detail.

More particularly, the actual capacitor in the biosensor 100 according to the exemplary embodiment of the invention is an electrolytic capacitor. The sensor 100 in this case is immersed in an electrolyte 150 during the measurement. The electrolyte 150 can be the analyte itself or another conducting fluid that replaces the analyte after capturing of the antigens by the immobilized capture probes 112 on the SAM surface. The copper nano-electrode 108 is one capacitor plate, the conducting fluid 150 is the other capacitor "plate". The two plates 108, 150 are separated by the SAM 115, which acts as the dielectric of the capacitor. When bio-molecules are attached to the SAM 115 (for instance as a result of the immobilization of the capture probes 112 on the SAM surface 115) or captured by the capture probes 112 (for instance as a result of the capturing of antigens by the capture probes 112) the dielectric properties of the capacitor's dielectric will change, and consequently also the capacitance of the capacitor. The electrolyte 150 is connected with the counter electrode 109.

As schematically indicated in Fig. 1, the transistor structure 113 is formed in the Front End of the Line portion 107 and is electrically coupled to the sensor active region 101 via the plurality of metallization structures 104 to 106, 108. A gate region 110 of such a transistor 113 is shown, as well as a channel region 111. Source/drain regions are located in front of and behind the plane of the drawing, respectively, and therefore are not indicated explicitly in Fig. 1. They may be formed as doped regions electrically coupled to both sides of the channel region 111, as known by the skilled person.

As can be taken from Fig. 1, a single antibody molecule 112 is immobilized at a surface 103 of the sensor active region 101 and is adapted for interacting with biological particles. Particularly, the antibody 112 is adapted for interacting with a corresponding antigen.

The copper metallization structure 108 may have, at the surface 103, a dimension of 250 nm and therefore forms a nanoelectrode at which a detection event may take place. The nanoelectrode 108 is formed of copper material lined with a tantalum nitride layer 114. As can further be taken from Fig. 1, a SAM layer 115 (self assembled monolayer) is bridging the copper structure 108 and the antibody 112.

The bare copper surface that remains after the final CMP step may oxidize rapidly in air or water. Therefore usually BTA (a corrosion inhibitor) is deposited during this CMP step (or during the subsequent cleaning step) to suppress this oxidation. In this way the wafers can be stored for some time (several days or perhaps even weeks) before the SAM 112 is deposited.

Just before the SAM deposition, the BTA is removed from the copper surface. Experimentally it is found that some wet-chemical SAM deposition recipes actually remove BTA themselves. In that case it is not strictly necessary to remove the BTA before the SAM deposition because it will happen automatically. After the SAM deposition it is not possible to deposit BTA anymore because the BTA would contaminate the SAM surface. Instead, a proper SAM 112 should act as a corrosion inhibitor by itself. Or the sensor chips have to be stored in a non-oxidizing atmosphere after the SAM deposition.

Beyond this, the biosensor chip 100 comprises an electrically insulating layer 116 forming part of a surface of the biosensor chip 100 and having a recess 117, wherein an exposed surface 103 of the sensor active region 101 is provided as a sensing pocket volume in the recess 117.

The biosensor chip 100 is manufactured in CMOS technology, starting from a silicon substrate 118, the surface of which is shown in Fig. 1, and which may have a P well or an N well.

Bond pads for electrically contacting the biosensor chip 100 may be provided but is not shown in Fig. 1.

More particularly, an electrically insulating shallow trench insulation structure 119 is provided on/in the semiconductor substrate 118. The gate 110 comprises polysilicon material and a CoSi silicide structure. Furthermore, a silicon carbide layer 120 is provided on the shallow trench insulation layer 119 and on the gate stack 110. A silicon oxide layer 121 has a contact hole in which the tungsten contact 106 is formed. On top of this structure, a further silicon carbide layer 141 is provided. On top of the silicon carbide layer 141, a tantalum nitride liner 122 is foreseen to line a trench, filled with copper material to form the copper metal structure 105. This is embedded in a further silicon oxide layer 123. On top of this structure, a further silicon carbide layer 124 is formed, followed by forming a tantalum nitride liner 125 in a via hole formed in a further silicon oxide layer 126. The lined via hole is filled with copper material, thereby forming the copper via 104. Next, a silicon carbide layer 127 may be deposited, followed by the position of a further silicon oxide layer 128, in which a further trench may be etched which may be lined with an additional tantalum nitride structure 129. This lined trench may be filled with copper material, thereby forming the copper metal layer 108.

A CMP (chemical mechanical polishing) procedure may be carried out to generate the essentially planar surface in the biosensor chip 100.

In the following, referring to Fig. 2, a biosensor chip 200 according to another embodiment of the invention will be explained. This embodiment is based on an etching procedure, as will be described below in more detail.

In contrast to the embodiment of Fig. 1, the embodiment of Fig. 2 has a further silicon oxide layer 201 on the silicon carbide layer 116, and an additional silicon carbide layer 202 on the silicon oxide layer 201. Further, an additional silicon oxide layer 203 is provided on the silicon carbide layer 202. A tantalum nitride structure 204 is provided as a word line, sandwiched between the layers 201, 202.

Again, a sensing pocket 117 is formed, and a bond pad is formed as well which is, however, not depicted in Fig. 2.

In Fig. 2, the capacitor is formed by the electrically conductive structures 108 and 204.

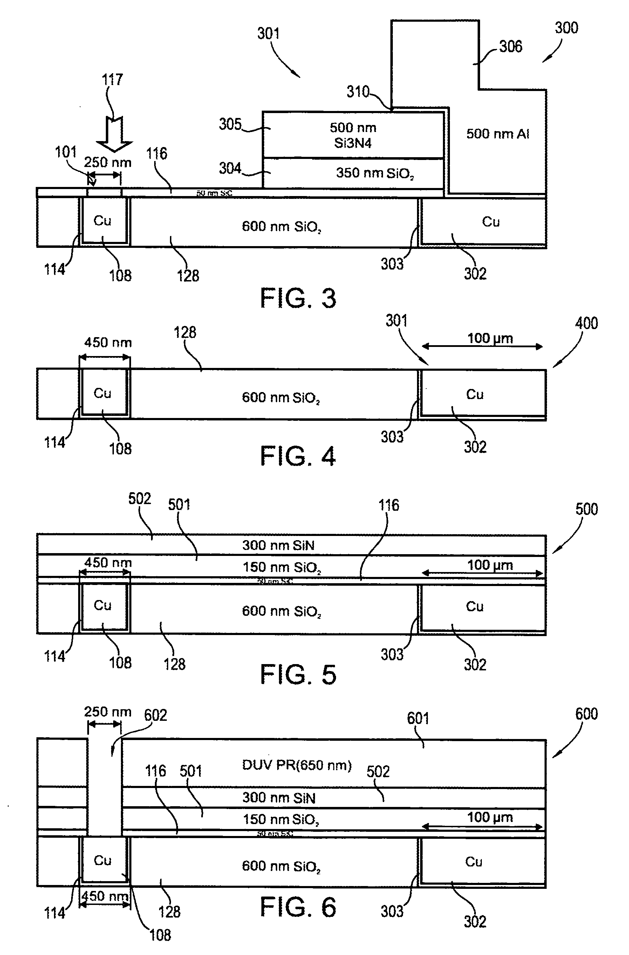

Fig. 3 shows a cross-sectional view 300 of a layer structure forming a biosensor chip according to an embodiment of the invention.

However, in Fig. 3, only the most upper portions are shown.

The sensing pocket 117 is shown again, as well as the sensor active surface 101.

In addition to the components shown, for instance, in Fig. 2 a bond pad region 301 is shown as well comprising a copper structure 302 formed in a trench lined with tantalum nitride 303, comprising a further silicon oxide layer 304, a silicon nitride layer 305 and an aluminium contact 306. A further tantalum nitride layer 310 is shown as well.

However, as will be described below referring to Fig. 4 to Fig. 11, the topography from patterning and the process complexity can be further reduced in this embodiment as compared to Fig. 3.

The manufacture procedure described referring to Fig. 4 to Fig. 11 starts at the "metal 2 layer" 108, as shown as a layer sequence 400 in Fig. 4.

As can be taken from Fig. 4, a lateral diameter of the copper structure 108 is 450 nm, whereas a dimension of a copper structure 302 of a bond pad area 301 has a dimension in the order of magnitude of 100 µm.

To obtain a layer sequence 500 shown in Fig. 5 starting from the layer sequence 400 shown in Fig. 4, a silicon carbide layer 116, a silicon oxide layer 501 and a silicon nitride layer 502 are deposited on the layer sequence 400, thereby performing a dielectric deposition after the "metal 2" 108 patterning.

To obtain a layer sequence 600 shown in Fig. 6, a photoresist layer 601 is formed on the layer structure 500, and a via etch procedure is carried out to thereby generate a hole 602.

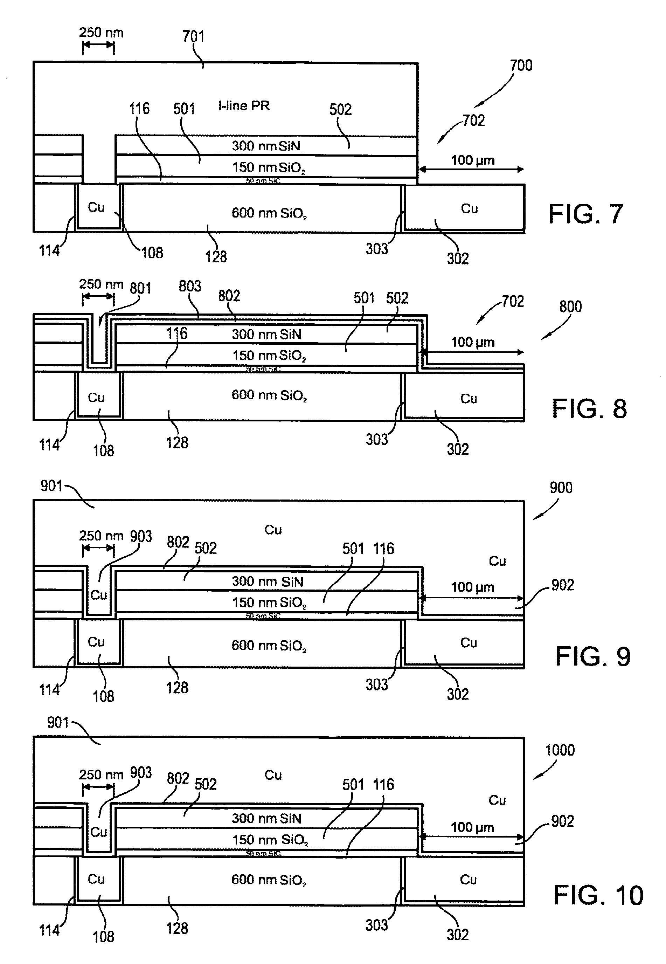

In order to obtain the layer sequence 700 shown in Fig. 7, the photoresist 601 is removed, and a photoresist layer 701 is deposited and patterned on the surface of the layer sequence 700, for the purpose of forming a bond pad opening 702.

In order to obtain a layer sequence 800 shown in Fig. 8, a next metal level deposition procedure is started. For this purpose, the photoresist 701 is removed from the surface as well as from the interior of the hole 602, that was filled at least partially by the photoresist 701, and a tantalum nitride liner 802 as well as a copper plating base 803 are deposited on top of the surface 800.

A layer sequence 900 shown in Fig. 9 is obtained by depositing copper material, thereby performing a copper plating procedure to generate the copper structure 901.

In the bond pad area 301, this generates a copper bond pad structure 902.

In the sensing region, this generates a second copper via 903.

To obtain a layer sequence 1000 shown in Fig. 10, a gas annealing is performed to improve the electrical and physical properties of the copper regions, and to remove plasma damage that may have accumulated during the etching steps.

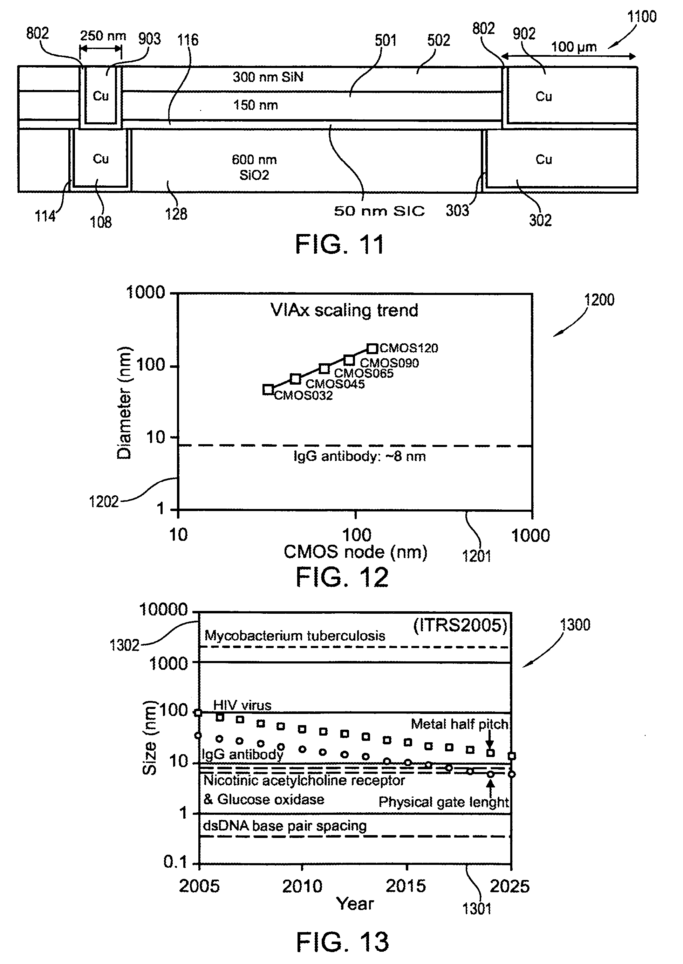

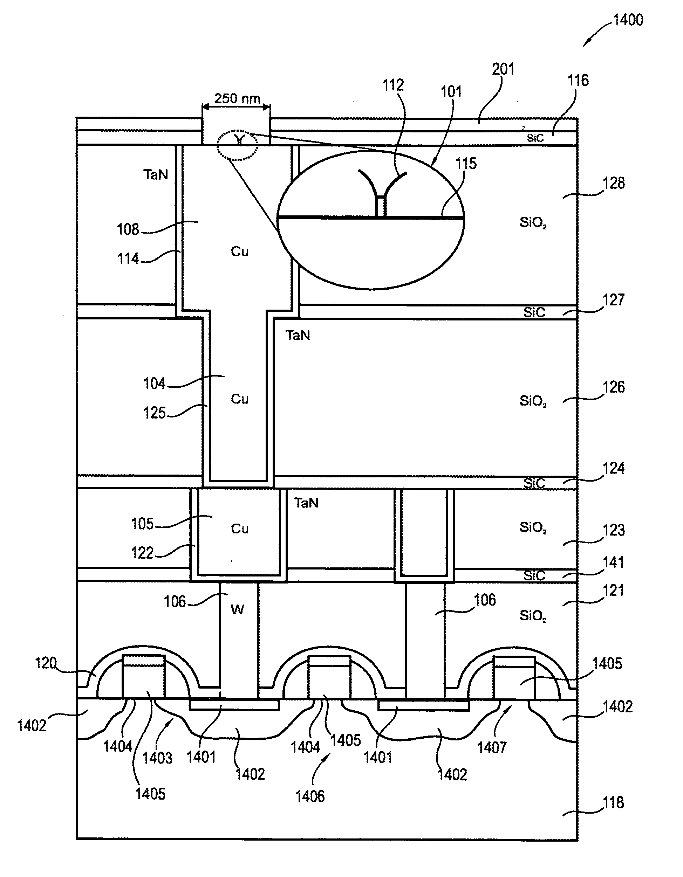

To obtain a layer sequence 1100 shown in Fig. 11, a CMP (chemical mechanical polishing) procedure is performed on the copper structure 901, thereby obtaining a planar topography by a simple process.

In the following, reasons will be explained why advanced CMOS technology is used for the biosensor chip according to the invention.

A main point is that minimum lithographic feature sizes in advanced CMOS technology are approaching the size of interesting biomolecules that are involved in processes related to disease diagnosis and monitoring. This fact may be used at the level of single molecule detection.

Fig. 12 shows a diagram 1200 having an abscissa 1201 along which a CMOS node is plotted in nm. Along an ordinate 1202 of the diagram 1200, a diameter in nm of CMOS structures is shown.

As can be taken from Fig. 12, there is a trend to lower dimensions in CMOS. As a comparison, the dimension of an IgG antibody is shown as a dashed line.

For comparison with Fig. 12, a diagram 1300 shown in Fig. 13 is plotted.

Along an abscissa 1301 the technology development is plotted on a time axis indicating a year. Along an ordinate 1302 of the diagram 1300, sizes of different structures are shown. As a comparison, the dimensions of the Mycobacterium Tuberculosis, the IgG antibody, the nicotinic acetylcholine receptor, the enzyme glucose oxidase, and the base pair spacing in a double-stranded DNA molecule are shown as dashed lines.

In other words, Fig. 12 and Fig. 13 show an example of how the minimum lithographic feature sizes of advanced CMOS semiconductor technology are approaching the sizes of biomolecules of interest in disease diagnosis and monitoring, and have reached already the sizes of bacteria and viruses. Fig. 12 in combination with Fig. 13 shows that the accuracy of the biosensor chip can be increased by implementing CMOS technologies of smaller and smaller minimum lithographic feature sizes.

Single molecule measurement (preferably done massively parallel) may give the additional possibility to extract much more information from a large number of nanoelectrodes with single molecule sensitivity than from one or a few larger electrodes that inherently only give signals related to average properties of ensembles of captured biomolecules.

It is possible to fabricate nanoelectrodes using conventional existing CMOS processing (for instance CMP and/or etching). Particularly, two appropriate ways of producing nanoelectrodes include etching and CMP procedures.

The etch approach allows for a very simple manufacture.

However, the CMP approach may be even more preferred, because apart from a lower process complexity and less topography, the CMP approach also results in a lower series resistance of the sensing nanoelectrode, caused by the spreading resistance in the electrolyte above the nanoelectrode surface. This is translated to a further improved signal-to-noise ratio and, consequently, improved sensitivity of the biosensing platform.

The nanoelectrodes may allow the detection of molecules related to certain diseases (for instance cancer) at the single molecular level. The single molecular range detection is achieved due to the dimensions of the electrodes that can be manufactured with CMOS.

Main advantages of the nanoelectrode approach according to the invention are the possibility of low-cost volume production in standard CMOS production facilities, the possibility to use standard design tools with conventional device models and calibrated parameters to design the embedded electronics for the sensing, signal-processing, control and input-output functions, easy scaling to next CMOS node generations, improvements of sensor performance (smaller nanoelectrodes), etc.

Copper nanoelectrodes are used as a material because copper is a default material available in the BEOL of CMOS. Copper has chemical similarities to gold (which has appropriate properties for electrical biosensors, but may be problematic when combined with semiconductor technology).

The deposited material is not limited to gold and can also include nickel or silver or silver chloride or other metals on to of the copper electrodes and formed for instance by electroplating or electroless plating.

Possible challenges of copper (such as oxidation) may be avoided or suppressed by protecting the copper surface with Benzotrialzole (BTA) that comes "for free" in a standard CMP process, and/or by depositing self-assembled monolayers (SAMS). However, it is also possible to deposit a thin gold layer self-aligned on the copper electrodes, for instance by electroplating or by a selective electroless deposition process.

Apart from the copper, there may also be a tantalum nitride diffusion barrier exposed at the surface. Advantageously, tantalum nitride can be anodically oxidized, thereby preventing direct contact with the electrolyte.

In the following, referring to Fig. 14, a biosensor chip 1400 according to an exemplary embodiment of the invention will be explained.

The biosensor 1400 differs from the previously described embodiments particularly regarding the transistor architecture.

Source/drain regions 1402 are provided as doped regions in the p well or n well 118. A channel region of the transistors is denoted with reference numeral 1404. A gate stack is denoted with reference numeral 1405. The tungsten contact 106 is coupled, via a metallically conductive structure 1401, to the source/drain regions 1402.

Therefore, when a sensor event takes place on the sensor active surface 101, this may cause a change of electrical properties which are transmitted via the structures 108, 104 to 106 to the source/drain region 1402 of the transistor 1403. Thus, when a gate voltage is applied to the gate 1405 of transistor 1403, the current flowing between the source/drain regions 1402 of transistor 1403 depends on the sensor event. A second transistor 1406 can be used to connect the nanoelectrode structure 101 via an alternative electrical connection path. A third transistor 1407 is part of an adjacent nanoelectrode detection cell.

The etched approach of Fig. 14 can be manufactured with low effort.

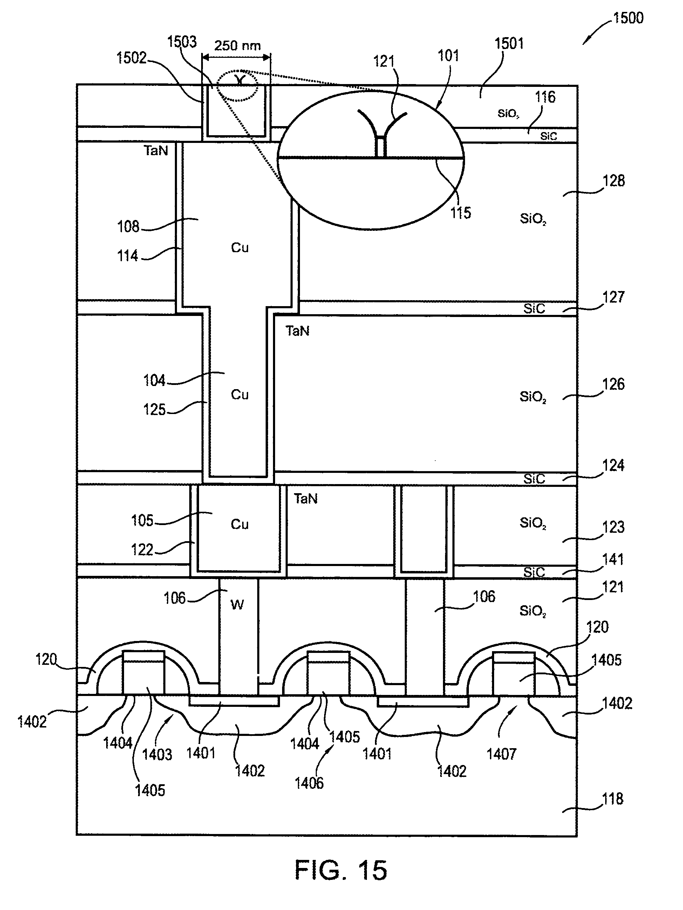

In the following, referring to Fig. 15, a biosensor chip 1500 according to an exemplary embodiment of the invention will be explained.

This may be manufactured using a CMP approach (chemical mechanical polishing). Fig. 15 shows a further silicon oxide layer 1501 with a via hole filled with a tantalum nitride liner 1502 and having a copper filling 1503.

## Claims

1. A biosensor chip (100) for detecting biological particles, the biosensor chip (100) adapted as a biosensor array and comprising a plurality of sensor active regions (101) that are sensitive for the biological particles, monolithically integrated in a common substrate (118), and arranged in a Back End of the Line Portion (102) of the biosensor chip (100), **characterized by**
each sensor active region (101) comprising a nanoelectrode (108) of copper material,
wherein an exposed copper surface of the sensor active region (101) has a diameter of at most 1.6 times of a minimum lithographic feature size of the smallest copper via holes of the CMOS process applied for manufacturing the biosensor chip (100).

2. The biosensor chip (100) of claim 1, wherein an exposed surface of the sensor active region (101) has a dimension of at most 1.1 times of said minimum lithographic feature size of a CMOS process applied for manufacturing the biosensor chip (100).

3. The biosensor chip (100) of claim 1, wherein an exposed surface of the sensor active region (101) has a dimension of at most 0.7 times of a minimum lithographic feature size of a CMOS process applied for manufacturing the biosensor chip (100).

4. The biosensor chip (100) of claim 1, wherein the sensor active region (101) is arranged at an upper surface (103) of a Back End of the Line portion (102) of the biosensor chip (100).

5. The biosensor chip (100) of claim 1, comprising at least one intermediate metallization structure (104 to 106), particularly at least one intermediate copper structure (104 to 106), in the Back End of the Line portion (102), wherein the sensor active region (101) is electrically coupled to a Front End of the Line portion (107) of the biosensor chip (100) via the at least one intermediate metallization structure (104 to 106).

6. The biosensor chip (100) of claim 1, comprising a capacitor structure (108, 109) at least partially formed in the Back End of the Line portion (102) and arranged such that a capacitance value of the capacitor (108, 109) is influencable by a detection event in the sensor active region (101).

7. The biosensor chip (100) of claim 1, comprising a switch transistor structure (113) formed in the Front End of the Line portion (107) and electrically coupled to the sensor active region (101).

8. The biosensor chip (100) of claim 1, comprising one or more capture molecules (112) arranged at a surface (103) of the sensor active region (101) and being adapted for interacting with the biological particles.

9. The biosensor chip (100) of claim 1, wherein an exposed surface (103) of the nanoelectrode (108) has a dimension of less than 300 nm.

10. The biosensor chip (100) of claim 1, wherein the nanoelectrode (108) comprises c copper material being covered by a self assembled monolayer.

11. The biosensor chip (100) of claim 1, comprising an electrically insulating layer (116) forming part of a surface of the biosensor chip (100) and having a recess (117), wherein an exposed surface (103) of the sensor active region (101) and the electrically insulating layer (116) form a sensing pocket in the recess (117).

12. The biosensor chip (100) according to claim 1, manufactured in CMOS technology, particularly manufactured in CMOS technology with an embedded option such as an embedded flask or an embedded DRAM

13. The biosensor chip (100) according to claim 1, wherein the substrate is a semiconductor substrate (118), comprising one of the group consisting of a group IV semiconductor, and a group III-group V semiconductor.

## Patentansprüche

1. Ein Biosensorchip (100) zum Detektieren von biologischen Partikeln, wobei der Biosensorchip (100) als ein Biosensor-Array eingerichtet ist und eine Mehrzahl von Sensor-aktiven Regionen (101) aufweist, die empfindlich auf die biologischen Partikel sind, monolithisch integriert in ein gemeinsames Substrat (118) sind und in einem Back End of the Line Teil (102) des Biosensorchip (100) angeordnet sind, **dadurch gekennzeichnet, dass**
jede Sensor-aktive Region (101) eine Nanoelektrode (108) aus Kupfermaterial aufweist,
wobei eine ausgesetzte Kupferoberfläche der Sensor-aktiven Region (101) einen Durchmesser von höchstens 1,6-mal einer minimalen lithographischen Merkmalsgröße des kleinsten Kupfervialochs des CMOS Verfahrens hat, welches zum Herstellen des Biosensorchips (100) angewendet wird.

2. Der Biosensorchip gemäß Anspruch 1, wobei eine ausgesetzte Kupferoberfläche der Sensor-aktiven Region (101) einen Durchmesser von höchstens 1,1-mal der minimalen lithographischen Merkmalsgröße eines CMOS Verfahrens hat, welches zum Herstellen des Biosensorchips (100) angewendet wird.

3. Der Biosensorchip gemäß Anspruch 1, wobei eine ausgesetzte Kupferoberfläche der Sensor-aktiven Region (101) einen Durchmesser von höchstens 0,7-mal einer minimalen lithographischen Merkmalsgröße eines CMOS Verfahrens hat, welches zum Herstellen des Biosensorchips (100) angewendet wird.

4. Der Biosensorchip (100) gemäß Anspruch 1, wobei die Sensor-aktive Region (101) an einer oberen Oberfläche (103) eines Back End of the Line-Teils (102) des Biosensorchips (100) angeordnet ist.

5. Der Biosensorchip (100) gemäß Anspruch 1, aufweisend zumindest eine Zwischenmetallisationsstruktur (104 bis 106), insbesondere zumindest eine Zwischenkupferstruktur (104 bis 106), in dem Back End of the Line-Teil (102), wobei die Sensor-aktive Region (101) elektrisch an einen Front End of the Line-Teil (107) des Biosensorchips über die zumindest eine Zwischenmetallisationsstruktur (104 bis 106) gekoppelt ist.

6. Der Biosensorchip (100) gemäß Anspruch 1, aufweisend eine Kondensatorstruktur (108, 109) die zumindest teilweise in dem Back End of the Line Teil (102) gebildet und so angeordnet ist, dass ein Kapazitätswert des Kondensators (108, 109) mittels eines Detektionsereignisses in der Sensor-aktiven Region (101) beeinflusst wird.

7. Der Biosensorchip (100) gemäß Anspruch 1, aufweisend eine Schalttransistorstruktur (113), die in dem Front End of the Line Teil (107) gebildet und elektrisch an die Sensor-aktive Region (101) gekoppelt ist.

8. Der Biosensorchip (100) gemäß Anspruch 1, aufweisend ein oder mehrere Fangmoleküle (112), die auf einer Oberfläche (103) der Sensor-aktiven Region (101) angeordnet und zum Wechselwirken mit den biologischen Partikeln eingerichtet sind.

9. Der Biosensorchip (100) gemäß Anspruch 1, wobei eine ausgesetzte Oberfläche (103) der Nanoelektrode (108) einen Durchmesser von weniger als 300 nm hat.

10. Der Biosensorchip (100) gemäß Anspruch 1, wobei die Nanoelektrode (108) Kupfermaterial aufweist, welches mittels einer selbstaufgebauten Monoschicht bedeckt wird.

11. Der Biosensorchip (100) gemäß Anspruch 1, aufweisend eine elektrisch isolierende Schicht (116), die Teil einer Oberfläche des Biosensorchips (100) bildet und die eine Ausnehmung (117) hat, wobei eine ausgesetzte Oberfläche (103) der Sensor-aktiven Region (101) und die elektrisch isolierende Schicht (116) eine Erkennaussparung in der Ausnehmung (117) bildet.

12. Der Biosensorchip (100) gemäß Anspruch 1, hergestellt in CMOS Technik, insbesondere hergestellt in CMOS Technik mit einer eingebetteten Option wie einen eingebetteten Behälter oder einen eingebetteten DRAM.

13. Der Biosensorchip (100) gemäß Anspruch 1, wobei das Substrat ein Halbleitersubstrat (118) ist, welches eines aus der Gruppe aufweist, bestehend aus einem Gruppe IV Halbleiter und einem Gruppe III-Gruppe V Halbleiter.

## Revendications

1. Puce de biocapteur (100) pour la détection de particules biologiques, la puce de biocapteur (100) étant agencée sous la forme d'une matrice de biocapteurs et comprenant une pluralité de régions actives de capteur (101) qui sont sensibles aux particules biologiques, intégrées de manière monolithique dans un substrat commun (118), et disposées lors de la partie terminale du process (BEOL) (102) de la puce de biocapteur (100), **caractérisée par le fait que**
chaque région active de capteur (101) comporte une nano-électrode (108) de matériau en cuivre,
dans laquelle une surface de cuivre exposée de la région active de capteur (101) a un diamètre égal à au moins 1,6 fois la dimension lithographique minimale des trous de via de cuivre dans le procédé CMOS appliqué à la fabrication de la puce de biocapteur (100).

2. Puce de biocapteur (100) selon la revendication 1, dans laquelle une surface exposée de la région active de capteur (101) a une dimension égale à au moins 1,1 fois ladite dimension lithographique minimale dans un procédé CMOS appliqué à la fabrication de la puce de biocapteur (100).

3. Puce de biocapteur (100) selon la revendication 1, dans laquelle une surface exposée de la région active de capteur (101) a une dimension égale à au moins 0,7 fois la dimension lithographique minimale dans le procédé CMOS appliqué à la fabrication de la puce de biocapteur (100).

4. Puce de biocapteur (100) selon la revendication 1, dans laquelle la région active de capteur (101) est disposée sur une surface supérieure (103) lors de la partie terminale du process (BEOL) (102) de la puce de biocapteur (100).

5. Puce de biocapteur (100) selon la revendication 1, comprenant au moins une structure de métallisation intermédiaire (104 à 106), particulièrement au moins une structure intermédiaire de cuivre (104 à 106), lors de la partie terminale du process (BEOL) (102), dans laquelle la région active de capteur (101) est raccordée électriquement à une partie de la phase initiale du process (FEOL) (107) de la puce de biocapteur (100) par l'intermédiaire d'au moins une structure de métallisation intermédiaire (104 à 106).

6. Puce de biocapteur (100) selon la revendication 1, comprenant une structure de condensateur (108, 109) formée au moins partiellement lors de la partie terminale du process (BEOL) (102) et disposée de telle manière que la valeur de la capacité du condensateur (108, 109) puisse être influencée par un événement de détection dans la région active du capteur (101).

7. Puce de biocapteur (100) selon la revendication 1, comprenant une structure de transistor de commutation (113) formée lors de la partie initiale du process (FEOL) (107) et raccordée électriquement à la région active du capteur (101).

8. Puce de biocapteur (100) selon la revendication 1, comprenant une ou plusieurs molécules de capture (112) disposées à la surface (103) de la région active du capteur (101) et qui sont agencées de manière à interagir avec les particules biologiques.

9. Puce de biocapteur (100) selon la revendication 1, dans laquelle une surface exposée (103) de la nano-électrode (108) a une dimension inférieure à 300 nm.

10. Puce de biocapteur (100) selon la revendication 1, dans laquelle la nano-électrode (108) comporte un matériau de cuivre recouvert d'une couche auto-assemblée mono-moléculaire.

11. Puce de biocapteur (100) selon la revendication 1, comprenant une couche isolante électrique (116) faisant partie d'une surface de la puce de biocapteur (100) et ayant une partie en retrait (117), dans laquelle une surface exposée (103) de la région active du capteur (101) et la couche isolante électrique (116) forment une poche de détection dans la partie en retrait (117).

12. Puce de biocapteur (100) selon la revendication 1, fabriquée dans une technologie CMOS, en particulier fabriquée dans une technologie CMOS avec une option intégrée comme une mémoire flash ou une mémoire DRAM intégrée.

13. Puce de biocapteur (100) selon la revendication 1, dans laquelle le substrat est un substrat de semi-conducteur (118) comprenant un élément parmi le groupe formé des semi-conducteurs du groupe IV et des semi-conducteurs du groupe III-V.
